(19) Europäisches Patentamt / European Patent Office / Office européen des brevets

(11) **EP 2 998 758 B1**

## (12) FASCICULE DE BREVET EUROPEEN

(45) Date de publication et mention de la délivrance du brevet:
**19.08.2020   Bulletin 2020/34**

(51) Int Cl.:
***G01R 33/24*** *(2006.01)*    *A61B 5/04* *(2006.01)*

(21) Numéro de dépôt: **15185192.0**

(22) Date de dépôt: **15.09.2015**

(54) **MAGNÉTOMÈTRE SANS ASSERVISSEMENT ET À COMPENSATION DES FLUCTUATIONS DE LA PENTE DE RESONANCE EN CHAMP FAIBLE, RÉSEAU DE MAGNÉTOMÈTRES ET PROCÉDÉ DE MESURE**

MAGNETOMETER OHNE REGELUNG UND MIT KOMPENSATION DER FLUKTUATIONEN DES RESONANZGEFÄLLES IN EINEM SCHWACHEN FELD, MAGNETOMETERNETZ UND MESSVERFAHREN

MAGNETOMETER WITHOUT SERVO CONTROL AND WITH COMPENSATION OF FLUCTUATIONS OF THE WEAK FIELD RESONANCE SLOPE, ARRAY OF MAGNETOMETERS AND MEASUREMENT METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **19.09.2014   FR 1458888**

(43) Date de publication de la demande:
**23.03.2016   Bulletin 2016/12**

(73) Titulaire: **Commissariat à l'Énergie Atomique et aux Énergies Alternatives
75015 Paris (FR)**

(72) Inventeurs:
- **LE PRADO, Matthieu
  38160 SAINT-MARCELLIN (FR)**
- **LEGER, Jean-Michel
  38190 VILLARD BONNOT (FR)**
- **MORALES, Sophie
  38760 VARCES (FR)**

(74) Mandataire: **Brevalex
95, rue d'Amsterdam
75378 Paris Cedex 8 (FR)**

(56) Documents cités:
**EP-A1- 0 463 919        EP-A1- 2 426 563
CA-A- 760 579             FR-A- 1 591 129
US-A- 2 975 360           US-A1- 2014 097 837
US-A1- 2014 121 491**

Remarques:
Le dossier contient des informations techniques présentées postérieurement au dépôt de la demande et ne figurant pas dans le présent fascicule.

**Description**

**DOMAINE TECHNIQUE**

Le domaine de l'invention est celui des magnétomètres pour lesquels la mesure d'un champ magnétique comprend la mesure de l'amplitude d'un signal à un harmonique d'une fréquence d'oscillation d'une source d'excitation. L'invention s'étend plus particulièrement aux réseaux de magnétomètres de type à noyau saturé ou de type atomique vectoriel, tels que ceux utilisés en magnétocardiographie ou en magnétoencéphalographie.

**ÉTAT DE LA TECHNIQUE ANTÉRIEURE**

On connaît des magnétomètres dont le principe de mesure est basé sur l'utilisation d'au moins un harmonique parmi plusieurs générés au moyen d'une source d'excitation.

C'est le cas par exemple des magnétomètres de type à noyau saturé (également connus sous le terme « fluxgate ») dans lesquels un courant d'excitation alternatif est appliqué à une bobine d'excitation entourant un noyau magnétique. Le champ magnétique à mesurer induit des pulsations de courant dans une bobine de détection qui entoure également le noyau magnétique, lesdites pulsations présentant des résonances à des harmoniques de la fréquence du courant d'excitation. L'amplitude des harmoniques paires est proportionnelle au champ à mesurer. La demande de brevet CA 760579 A décrit un magnétomètre de ce type.

C'est également le cas des magnétomètres atomiques vectoriels. Ces magnétomètres emploient une cellule remplie d'un gaz d'atomes, une source laser qui émet un faisceau polarisé en direction de la cellule selon une direction de propagation, ainsi qu'un photodétecteur apte à délivrer un signal de sortie représentatif du faisceau ayant traversé la cellule. Une bobine entoure la cellule, alimentée par un générateur de fréquence pour générer un champ magnétique d'excitation sinusoïdal, perpendiculaire à ladite direction de propagation et parallèle au champ à mesurer.

On pourra trouver une présentation du principe de fonctionnement de tels magnétomètres atomiques dans l'article de J. Dupont-Roc, intitulé «Etude théorique de diverses résonances observables en champ nul sur des atomes "habillés" par des photons de radiofréquence», Le journal de physique, Tome 32, février 1971, p135.

Le signal S capté par le photodétecteur comprend plusieurs harmoniques dont les expressions sont les suivantes.

$$S = J_0 M + \sum_{q \geq 1} (2 J_{2q} \cos 2q\omega t).M - \sum_{q \geq 0} (2 J_{2q+1} \cos(2q+1)\omega t).N \text{ ,}$$

où

$$M = \frac{\lambda J_0}{\Gamma} \frac{\Gamma^2 + \omega_x{}^2}{\Gamma^2 + \omega_x{}^2 + \omega_y{}^2 + \omega_z{}^2} \text{ ,}$$

$$N = \frac{\lambda J_0}{\Gamma} \frac{\Gamma \omega_z + \omega_x \omega_y}{\Gamma^2 + \omega_x{}^2 + \omega_y{}^2 + \omega_z{}^2} \text{ ,}$$

$\omega_i$ est le champ magnétique sur l'axe i, multiplié par y le rapport gyromagnétique du niveau d'énergie considéré pour l'atome,
$\lambda$ décrit l'orientation induite par le pompage optique,
$1/\Gamma$ est le temps de relaxation du niveau d'énergie considéré pour l'atome,

$J_n$ est la fonction de Bessel d'ordre n et d'argument $\dfrac{\gamma B_1}{\omega}$ .

S contient plus particulièrement des résonances impaires aux fréquences $\dfrac{(2q+1)\omega}{2\pi}$ (avec q un entier positif ou nul) qui sont proportionnelles au champ magnétique à mesurer sur l'axe z.

Seule la résonance à la fréquence du champ d'excitation $\omega/2\pi$ est en réalité traitée, les autres harmoniques n'étant pas utilisés. Cette résonance est présente en champ magnétique faible $\omega_i < \Gamma$, où $i \in \{x, y, z\}$. Elle permet de mesurer un champ magnétique faible via l'asservissement d'un champ magnétique de compensation Bc dont l'amplitude est ajustée afin que la somme $B_C + B_0$ soit maintenue à zéro en permanence. De cette manière, la connaissance du courant Ic injecté dans la bobine pour appliquer le champ de compensation permet de connaître le champ magnétique ambiant $B_0$ puisque Bc = -$B_0$.

Les magnétomètres asservis en champs magnétique nul exploitent ainsi des signaux résonants observables en champ magnétique très faible. Le principe de fonctionnement est comme on l'a vu ci-dessus de générer un champ magnétique de compensation $B_C$, qui vient s'opposer au champ magnétique ambiant $B_0$. Dans le cadre d'un réseau de magnétomètres, le champ magnétique de compensation $B_C$ généré par les bobines d'un magnétomètre est également vu par les autres magnétomètres du réseau, les plus proches en particulier. Il vient perturber les mesures effectuées par les autres magnétomètres du réseau et peut également conduire à des instabilités des magnétomètres.

En pratique, les magnétomètres de types fluxgate ou atomiques vectoriels sont séparés de plusieurs centimètres, lorsqu'ils sont déployés en réseau, pour ne pas se perturber entre eux. Mais un tel écartement ne peut être adopté dans des applications telles que la magnétocardiographie ou la magnétoencéphalographie où le pas du réseau peut être aussi petit que 1 à 2 centimètres.

## EXPOSÉ DE L'INVENTION

L'invention a pour objectif de proposer une solution autre que l'écartement des capteurs du réseau pour limiter les interférences dans un réseau de magnétomètres. Elle propose à cet effet un dispositif de mesure d'une composante d'un champ magnétique conforme à la revendication 1.

Certains aspects préférés mais non limitatifs de ce dispositif sont définis dans les revendications dépendantes 2-7.

L'invention porte également sur un appareil de mesure du champ magnétique comprenant une pluralité de dispositifs selon le premier aspect de l'invention agencés en réseau. Dans un tel appareil, la source d'excitation secondaire d'un dispositif peut être propre audit dispositif. Il peut également comprendre une source d'excitation secondaire commune auxdits dispositifs. L'invention concerne également un procédé de mesure d'une composante de champ magnétique tel que défini par la revendication 13.

## BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :

- la figure 1 illustre le principe de l'asservissement en champ magnétique nul mis en œuvre avec les magnétomètres de l'art antérieur ;
- la figure 2 illustre la portion linéaire de la courbe de résonance observable en présence d'un champ magnétique faible ;
- la figure 3 illustre le principe de mesure sans asservissement en champ magnétique nul mis en œuvre avec les magnétomètres conformes à l'invention ;
- la figure 4 est un schéma d'un magnétomètre atomique à résonances paramétriques en champ nul conforme à un mode de réalisation possible de l'invention.
- la figure 5 illustre des signaux de référence employés dans un réseau de magnétomètres en boucle ouverte selon un premier mode de réalisation possible de l'invention ;
- la figure 6 illustre des signaux de référence employés dans un réseau de magnétomètres en boucle ouverte selon un second mode de réalisation possible de l'invention.

## EXPOSÉ DÉTAILLÉ DE MODES DE RÉALISATION PARTICULIERS

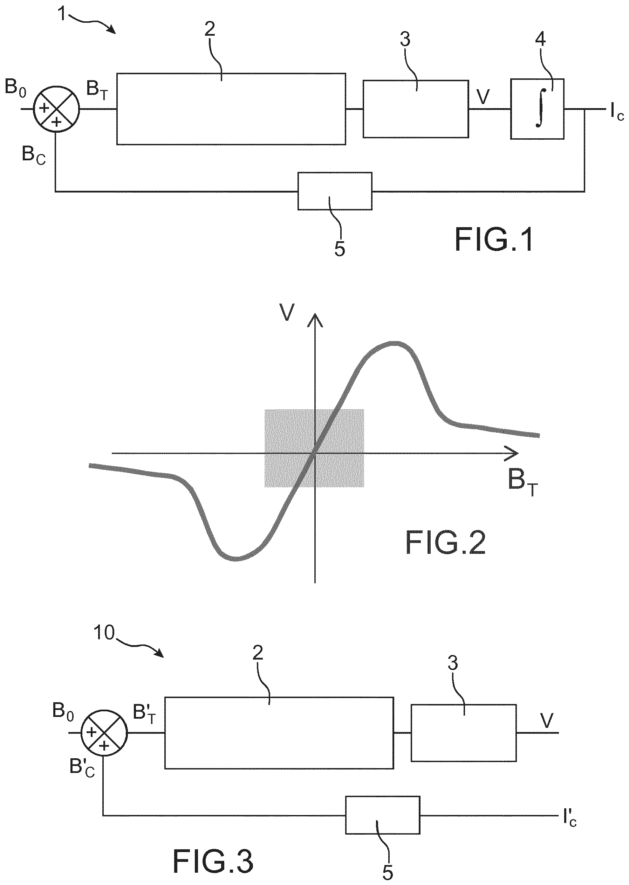

L'asservissement en champ nul représenté sur la figure 1 est traditionnellement mis en œuvre pour réaliser la mesure du champ magnétique $B_0$. Le dispositif de mesure 1 comprend une source d'excitation 2 permettant la résonance, par exemple une bobine entourant la cellule et alimentée par un générateur de fréquence dans le cas d'un magnétomètre vectoriel atomique, et un détecteur 3 permettant de mesurer l'amplitude d'un signal de sortie à un harmonique de la fréquence d'oscillation de la source d'excitation.

Cette amplitude, et la valeur de courant calculée à partir de cette amplitude au moyen d'un intégrateur 4, s'annulent pour un champ magnétique total $B_T$ nul. On applique pour ce faire le champ magnétique de compensation

$B_C$ au moyen d'une bobine 5 alimentée par un courant $I_C$. La mesure du courant $I_C$ injecté dans la bobine 5 permet de connaître, via la fonction de transfert courant/champ magnétique de la bobine, la valeur du champ magnétique de compensation $B_C$ et donc celle du champ ambiant $B_0$.

L'asservissement décrit en référence à la figure 1 est nécessaire pour chaque axe de mesure des magnétomètres. Il faut ainsi trois asservissements de ce type, afin de mesurer les trois composantes du champ ambiant $B_0$.

On a représenté sur la figure 2 la courbe de résonance représentant l'amplitude V du signal mesuré par le détecteur 3 en fonction du champ magnétique total $B_T$. Cette courbe présente une portion linéaire lorsque le champ magnétique est faible et au voisinage de laquelle l'amplitude est directement proportionnelle au champ magnétique.

Prenant en considération le fait que l'amplitude des signaux magnétiques issus du cœur ou du cerveau est inférieure à 1 nT, soit très inférieure à l'étendue de la portion linéaire de la courbe de résonance, qui est de quelques dizaines de nT pour un magnétomètre atomique vectoriel et de quelques centaines de µT pour un magnétomètre de type fluxgate, l'invention propose de ne pas mettre en œuvre l'asservissement en champ magnétique nul mais de réaliser la mesure du champ magnétique ambiant $B_0$ directement via la mesure de l'amplitude V de la résonance et grâce à la connaissance de la pente de la portion linéaire de la courbe de résonance.

On a représenté sur la figure 3 le principe de mesure sans asservissement en champ nul mis en œuvre avec les magnétomètres de l'invention. On retrouve dans le dispositif de mesure 10 selon l'invention la source d'excitation 2 et le détecteur 3 précédemment décrit.

Dans un mode de réalisation possible représenté sur la figure 3, le champ ambiant $B_0$ peut être préalablement compensé, en particulier si celui-ci est trop important pour garantir une utilisation des résonances sur leur portion linéaire. A cet effet, un courant constant $I'_C$ est injecté dans la bobine 5 pour générer un champ de compensation constant $B'_C$ tel que le champ mesuré $B'_T = B_0 + B'_C$ soit suffisamment faible pour garantir une résonance dans la portion linéaire.

On comprendra que dans le cadre de l'invention, le champ magnétique à mesurer est un champ dont l'amplitude garantit une résonance dans la portion linéaire. Il peut s'agir du champ ambiant $B_0$ (par exemple pour des signaux magnétiques issus du cœur ou du cerveau) ou du champ ambiant compensé $B'_T$ comme mentionné ci-dessus pour garantir une résonance dans la portion linéaire.

Ainsi, l'invention a trait à un dispositif 10 de mesure d'une composante d'un champ magnétique comportant un détecteur 3 configuré pour mesurer l'amplitude V d'un signal de sortie à une fréquence d'oscillation d'une source d'excitation 2 et pour en déduire le champ magnétique $B'_T$ à mesurer à partir de ladite amplitude et de la valeur d'une pente de résonance caractéristique d'une relation de proportionnalité entre ladite amplitude et la composante du champ magnétique à mesurer (champ $B_0$ ou $B'_T$ selon qu'une compensation soit nécessaire pour obtenir cette relation de proportionnalité).

En l'absence d'asservissement en champ nul, on supprime les interférences entre magnétomètres d'un réseau de magnétomètres. Toutefois, la pente de résonance, qui permet de remonter au champ magnétique à partir de la mesure de l'amplitude de la résonance, dépend de plusieurs paramètres qui varient dans le temps. Il s'agit par exemple de la puissance du laser, de la température, des déplacements des fibres optiques pour le magnétomètre atomique vectoriel. Au sein d'un réseau par ailleurs, les pentes des résonances propres à chacun des magnétomètres peuvent varier significativement et indépendamment les unes des autres et conduire à des erreurs dans les algorithmes de reconstruction de source magnétique utilisés par exemple en magnétocardiographie ou en magnétoencéphalographie.

Afin de contrôler l'impact de l'évolution de la pente de résonance, l'invention propose une technique permettant de calibrer les magnétomètres. Selon cette technique, un signal de référence d'amplitude connue et suffisamment faible relativement à l'étendue de la portion linéaire de la courbe de résonance est imposé à un magnétomètre. L'amplitude de la résonance induite par ce signal de référence permet de remonter à la valeur de la pente de résonance.

L'invention propose ainsi un dispositif de mesure d'un champ magnétique tel que précédemment décrit et caractérisé en ce qu'il comporte une source d'excitation principale fournissant un signal de mesure oscillant à une fréquence d'oscillation principale et une source d'excitation secondaire fournissant un signal de référence d'amplitude connue oscillant à une fréquence d'oscillation secondaire différente de la fréquence d'oscillation principale, le détecteur étant en outre configuré pour mesurer l'amplitude du signal de sortie à un harmonique de la fréquence d'oscillation secondaire et pour en déduire ladite pente de résonance à partir de ladite amplitude du signal de sortie à un harmonique de la fréquence d'oscillation secondaire et de l'amplitude connue du signal de référence.

Connaissant la pente de résonance, le détecteur peut alors déduire la valeur de la composante du champ à mesurer à partir de la mesure de l'amplitude du signal de sortie à un harmonique de la fréquence d'oscillation principale.

Le dispositif peut en outre comprendre une source d'excitation tertiaire fournissant un signal de compensation d'amplitude constante oscillant à la fréquence d'oscillation principale. Cette source permet de générer un champ de compensation $B'_C$ constant.

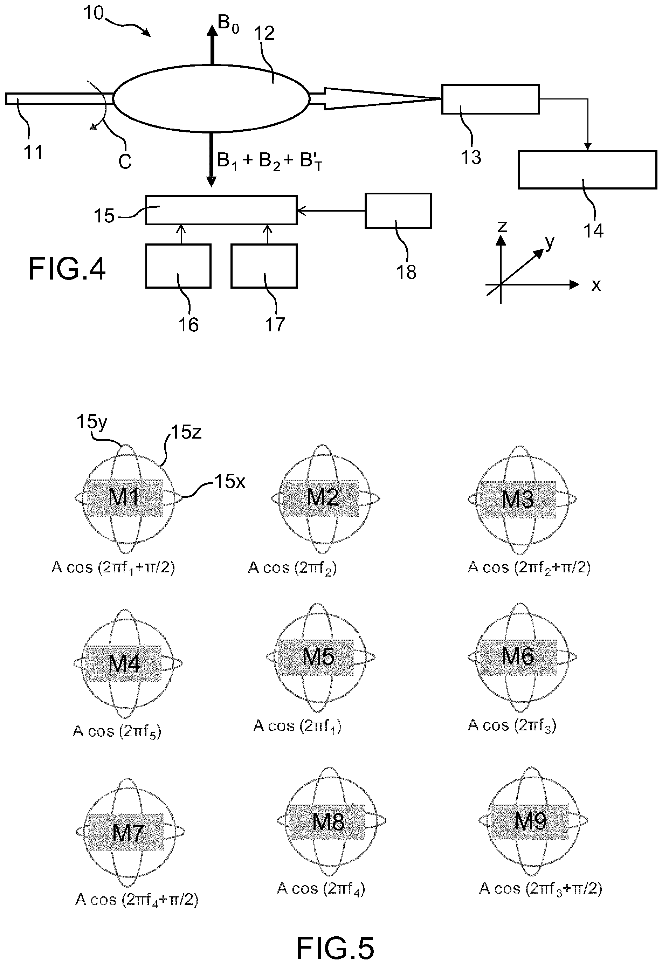

On a représenté sur la figure 4 un schéma d'un magnétomètre atomique à résonances paramétriques en champ nul conforme à un mode de réalisation possible de l'invention. Le magnétomètre comprend un laser émettant un faisceau 11, un polariseur circulaire C délivrant un faisceau polarisé circulairement, une cellule 12 remplie par exemple d'hélium 4, un photodétecteur 13 recevant le faisceau ayant traversé la cellule 12, un détecteur synchrone 14 réglé d'une part à un harmonique de la fréquence d'oscillation principale et, d'autre part, à un harmonique de la fréquence d'oscillation secondaire.

Le magnétomètre comprend par ailleurs des bobines 15 alimentées par des générateurs de fréquence 16, 17, 18. Un premier générateur de fréquence 16 permet de générer le champ magnétique d'excitation principal B1 (champ de mesure), un deuxième générateur de fréquence 17 permet de générer le champ magnétique d'excitation secondaire B2 d'amplitude connue (champ de référence) et, dans une variante de réalisation possible, un troisième générateur de fréquence 18 permet, comme discuté précédemment, de générer un champ magnétique de compensation $B'_C$ constant.

Dans le cadre de l'invention, le détecteur est plus précisément configuré pour déterminer le champ magnétique de référence B2 généré par la source d'excitation secondaire en exploitant une fonction de transfert de la source d'excitation reliant l'amplitude au champ magnétique.

La source d'excitation secondaire peut notamment comprendre au moins une bobine 7, généralement une pluralité de bobines chacune associée à un axe du magnétomètre, et un générateur de fréquence 17 permettant d'injecter un courant d'amplitude connue dans la bobine. Or la fonction de transfert courant/champ magnétique d'une bobine équipant un magnétomètre fluxgate ou atomique vectoriel peut être déterminée avec précision, par exemple avec la méthode décrite dans le document EP2426563A1 pour un magnétomètre atomique.

Dans un premier mode de réalisation de l'invention, tel que représenté sur la figure 4, la source d'excitation secondaire utilise les bobines déjà présentes sur chaque magnétomètre. Le signal de référence d'amplitude connue est utilisé pour appliquer un champ magnétique dans une bande de fréquence disjointe de signaux d'intérêt pour l'application (de 0 à 0.1Hz, ou au-delà de 100 Hz pour la magnétocardiographie ou la magnétoencéphalographie), et permet de connaitre en temps réel la valeur de la pente de la résonance associée à la bobine qui génère le champ magnétique via la mesure de l'amplitude de la résonance à un harmonique de la fréquence d'oscillation secondaire.

Cette approche est possible pour tous les axes d'un magnétomètre (pour remonter à toutes les composantes du champ magnétique à mesurer), et pour tous les magnétomètres d'un réseau le cas échéant.

Dans une première variante, la source d'excitation secondaire est configurée pour fournir un même signal de référence tour à tour et individuellement sur chacun desdits axes.

Dans une deuxième variante permettant notamment une calibration temps réel, la source d'excitation secondaire est configurée pour fournir un signal de référence simultanément sur chacun desdits axes, le signal de référence fourni à un axe présentant une fréquence d'oscillation différente de celle du signal de référence fourni à un autre axe, ou étant en quadrature de phase avec le signal de référence fourni à un autre axe. Il est en effet nécessaire que les signaux de référence vus par un magnétomètre ne se superposent pas aux signaux de référence générés par les magnétomètres voisins. Les signaux de référence doivent alors être « orthogonaux », soit à des fréquences différentes ou en quadrature de phase, par exemple, pour réduire le nombre de fréquences employées. On a à cet égard représenté sur la figure 5 des exemples de signaux de référence employés dans un réseau de neuf magnétomètres M1-M9 en boucle ouverte selon cette deuxième variante du premier mode de réalisation possible de l'invention. Des signaux de référence en quadrature de phase sont ici utilisés pour limiter à cinq le nombre de fréquences f1-f5 employées. Pour chacun des magnétomètres, on a représenté les bobines 15x, 15y, 15z permettant d'appliquer un signal de référence sur chacun des trois axes.

On notera que ce réseau de neuf magnétomètres est répétable. Il permet ainsi aux plus proches voisins de ne pas se perturber. Cette technique peut également prendre en compte le cas des voisins du deuxième rang. De nouvelles fréquences, ou en tous cas d'autres signaux de références orthogonaux à ceux de la première série, doivent pour ce faire être utilisés. Et on notera que cette technique peut également être mise en œuvre dans un réseau de magnétomètres développés sur trois dimensions et non sur deux dimensions comme dans l'exemple de la figure 5.

Ainsi l'invention s'étend également à un appareil de mesure du champ magnétique comprenant une pluralité de dispositifs de mesure tels que précédemment décrits qui sont agencés en réseau, appareil dans lequel la source d'excitation secondaire d'un dispositif est propre audit dispositif. Les sources d'excitation secondaires desdits dispositifs peuvent notamment être configurées pour fournir ledit signal de référence tour à tour à chacun des dispositifs. Et la source d'excitation secondaire et la source d'excitation principale d'un dispositif comprennent de préférence au moins une bobine en commun.

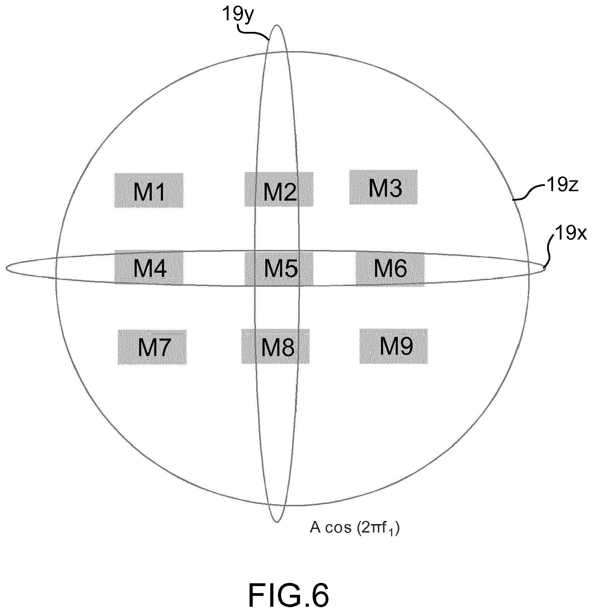

Dans un second mode de réalisation de l'invention, tel que représenté sur la figure 6, le circuit d'excitation secondaire est configuré pour générer un signal de référence commun à tous les magnétomètres au moyen de bobines conçues pour produire un champ magnétique « commun » à l'échelle du réseau. L'invention s'étend ainsi à un appareil de mesure du champ magnétique comprenant une pluralité de dispositifs M1-M9 tels que précédemment décrits qui agencés en réseau, appareil dans lequel la source d'excitation secondaire est commune auxdits dispositifs. La source d'excitation secondaire peut notamment comprendre au moins une bobine 19x, 19y, 19z englobant l'appareil M1-M9 et un générateur de fréquence permettant d'injecter un courant d'amplitude connue dans la bobine. Une fréquence différente peut être adoptée pour chacun des axes.

Ce second mode de réalisation présente l'avantage de permettre une calibration des défauts d'alignement des magnétomètres du réseau. Il souffre en revanche de la nécessité de rajouter un ensemble supplémentaire au réseau et donc d'un encombrement et d'un coût plus importants.

La description suivante a trait à un exemple de réalisation de l'invention dans lequel les magnétomètres du réseau sont des magnétomètres atomiques vectoriels. Les pentes de leurs résonances sont de 10 mV/nT sur les axes X et Y et de 3 mV/nT pour l'axe Z. La partie linéaire des résonances est limitée à $\pm$ 5 nT. Les éléments sensibles des magnétomètres sont des parallélépipèdes rectangles de 2 cm de côté. Le pas du réseau surfacique de magnétomètres est de 5 mm. Il est composé de neuf magnétomètres distribués comme sur les figures 5 et 6. Le champ magnétique à l'emplacement du réseau est de [0, 0, 10] nT sur les axes [X, Y, Z].

Dans le cadre d'une mise en œuvre du premier mode de réalisation de l'invention, La géométrie des bobines permet de générer des champs magnétiques homogènes. Cette géométrie, décrite dans l'article de J.C. Alldred et al. intitulé "Square cross section coils for the production of uniform magnetic fields", J. Sci. Instrum., 1967, Vol. 44, prend ici pour paramètres $a_1$ = 0.955, $a_2$ = 1.000, $h_1$ = 1.051, $h_2$ = 0.288, $N_1$ = 21/11, $N_2$ = 1.

Le champ magnétique généré vaut B = 100 x 1.83 $10^{-4}$ T / A, avec une homogénéité inférieure à $10^{-3}$ dans une sphère de 1 cm de rayon. L'homogénéité du champ généré impacte directement l'incertitude sur la fonction de transfert en boucle ouverte qui sera établie en fin de processus et pour laquelle on vise une connaissance à $10^{-2}$ près. L'architecture des bobines choisie est donc confortable vis-à-vis de cet objectif.

Les fonctions de transfert des bobines sont donc de 18.3 nT/$\mu$A, pour chacun des 3 axes des magnétomètres. Le magnétomètre au centre du réseau est dans un premier temps asservi en champ nul. Il mesure le champ magnétique de [0, 0, 10] nT et le compense avec des courants de [0, 0, 0.54] $\mu$A injectés dans ses bobines. Les courants appliqués sont maintenus mais les asservissements sont stoppés.

L'application visée est de la magnétocardiographie. L'énergie de la signature magnétique des battements du cœur est essentiellement au-delà de 0.5 Hz. On choisit des valeurs 0.40, 0.35, 0.30, 0.25 et 0.20 Hz pour les fréquences $f_1$, $f_2$, $f_3$, $f_4$ et $f_5$, définies d'après la figure 5. Les valeurs des phases sont également celles de la figure 5.

On choisit pour les signaux de référence, sinusoïdaux, la même amplitude correspondant à 1 nT, soit des courants de 0.054 $\mu$A en entrée de bobines.

Dans le cadre d'une mise en œuvre du second mode de réalisation de l'invention, les bobines employées ont la même architecture que celles du premier mode de réalisation mais avec des côtés de 2m afin de contenir le réseau de magnétomètres et le patient. Leur fonction de transfert vaut 1.83 $10^{-4}$ T / A. On choisit pour les signaux de référence, sinusoïdaux, la même fréquence, fi = 0.2 Hz par exemple, et la même amplitude correspondant à 1 nT, soit des courants de 5.4 $\mu$A en entrée de bobines.

Dans l'un et l'autre de ces modes de réalisation, on procède à une détection synchrone par axe de magnétomètre pour quantifier l'impact en boucle ouverte du signal de référence. La tension mesurée au niveau des détections synchrones évolue en temps réel (les pentes des résonances ne sont pas constantes dans le temps) dans la plage [10$\pm$1, 10$\pm$1, 3$\pm$0.5] mV sur les axes X, Y, Z respectivement pour des références dont l'amplitude est de 1 nT. Elle permet ainsi de connaitre l'évolution des pentes des axes des magnétomètres de [10$\pm$1, 10$\pm$1, 3$\pm$0.5] mV/nT au cours de la mesure et ainsi la fonction de transfert du magnétomètre opéré en boucle ouverte.

L'invention n'est pas limitée à un magnétomètre ou à un réseau de magnétomètres tels que précédemment décrit mais s'étend également à un procédé de mesure d'une composante d'un champ magnétique réalisé par un tel magnétomètre ou réseau de magnétomètre, et en particulier à un procédé mettant en œuvre une mesure de l'amplitude d'un signal de sortie à une fréquence d'oscillation d'une source d'excitation et une détermination de la composante du champ magnétique à mesurer à partir de la valeur d'une pente de résonance caractéristique d'une relation de proportionnalité entre ladite amplitude et la composante du champ magnétique à mesurer, le procédé étant caractérisé par la superposition à un signal de mesure oscillant à la fréquence d'oscillation principale d'un signal de référence d'amplitude connue oscillant à une fréquence d'oscillation secondaire différente de la fréquence d'oscillation principale, par la mesure de l'amplitude du signal de sortie à un harmonique de la fréquence d'oscillation secondaire et par la détermination de ladite pente de résonance à partir de ladite amplitude du signal de sortie à un harmonique de la fréquence d'oscillation secondaire et de l'amplitude connue du signal de référence.

Dans l'un et l'autre de ces modes de réalisation, on procède à une détection synchrone par axe de magnétomètre pour quantifier l'impact en boucle ouverte du signal de référence. La tension mesurée au niveau des détections synchrones évolue en temps réel (les pentes des résonances ne sont pas constantes dans le temps) dans la plage [10$\pm$1, 10$\pm$1, 3$\pm$0.5] mV sur les axes X, Y, Z respectivement pour des références dont l'amplitude est de 1 nT. Elle permet ainsi de connaitre l'évolution des pentes des axes des magnétomètres de [10$\pm$1, 10$\pm$1, 3$\pm$0.5] mV/nT au cours de la mesure et ainsi la fonction de transfert du magnétomètre opéré en boucle ouverte.

L'invention n'est pas limitée à un magnétomètre ou à un réseau de magnétomètres tels que précédemment décrit mais s'étend également à un procédé de mesure d'une composante d'un champ magnétique réalisé par un tel magnétomètre ou réseau de magnétomètre, et en particulier à un procédé mettant en œuvre une mesure de l'amplitude d'un signal de sortie à une fréquence d'oscillation d'une source d'excitation et une détermination de la composante du champ magnétique à mesurer à partir de la valeur d'une pente de résonance caractéristique d'une relation de proportionnalité entre ladite amplitude et la composante du champ magnétique à mesurer, le procédé étant caractérisé par la superposition à un signal de mesure oscillant à la fréquence d'oscillation principale d'un signal de référence d'amplitude connue oscillant à une fréquence d'oscillation secondaire différente de la fréquence d'oscillation principale, par la mesure de l'amplitude du signal de sortie à un harmonique de la fréquence d'oscillation secondaire et par la détermination de ladite pente de résonance à partir de ladite amplitude du signal de sortie à un harmonique de la fréquence d'oscillation secondaire et de l'amplitude connue du signal de référence.

**Revendications**

1. Dispositif (10) de mesure d'une composante d'un champ magnétique comportant un détecteur (3, 13, 14) configuré pour mesurer l'amplitude d'un signal de sortie à une fréquence d'oscillation d'une source d'excitation et pour en déduire la composante du champ magnétique à mesurer à partir de la valeur d'une pente de résonance caractéristique d'une relation de proportionnalité entre ladite amplitude et la composante du champ magnétique à mesurer, **caractérisé en ce qu'**il comporte une source d'excitation principale fournissant un signal de mesure (B1) oscillant à une fréquence d'oscillation principale et une source d'excitation secondaire fournissant un signal de référence d'amplitude connue (B2) oscillant à une fréquence d'oscillation secondaire différente de la fréquence d'oscillation principale, et **en ce que** le détecteur est en outre configuré pour mesurer l'amplitude (V) du signal de sortie à un harmonique de la fréquence d'oscillation secondaire et pour en déduire la valeur de ladite pente de résonance à partir de ladite amplitude du signal de sortie à un harmonique de la fréquence d'oscillation secondaire et de l'amplitude connue du signal de référence.

2. Dispositif selon la revendication 1, dans lequel le détecteur est configuré pour déterminer un champ magnétique de référence généré par la source d'excitation secondaire en exploitant une fonction de transfert de la source d'excitation secondaire reliant l'amplitude à la composante du champ magnétique.

3. Dispositif selon la revendication 2, dans lequel la source d'excitation secondaire comprend au moins une bobine (15) et un générateur de fréquence (17) permettant d'injecter un courant d'amplitude connue dans la bobine.

4. Dispositif selon la revendication 3, dans lequel la source d'excitation secondaire comprend une pluralité de bobines (15x, 15y, 15z ; 19x, 19y, 19z) chacune associé à un axe du dispositif.

5. Dispositif selon la revendication 4, dans lequel la source d'excitation secondaire est configurée pour fournir un signal de référence tour à tour sur chacun desdits axes.

6. Dispositif selon la revendication 5, dans lequel la source d'excitation secondaire est configurée pour fournir un signal de référence simultanément sur chacun desdits axes, le signal de référence fourni à un axe présentant une fréquence d'oscillation différente de celle du signal de référence fourni à un autre axe, ou étant en quadrature de phase avec le signal de référence fourni à un autre axe.

7. Dispositif selon l'une des revendications 1 à 6, comprenant en outre une source d'excitation tertiaire (15, 18) fournissant un signal de compensation (B'c) d'amplitude constante oscillant à la fréquence d'oscillation principale.

8. Appareil de mesure d'une composante d'un champ magnétique comprenant une pluralité de dispositifs (M1-M9) selon l'une des revendications 1 à 7 agencés en réseau, dans lequel la source d'excitation secondaire (15, 17, 15x, 15y, 15z) d'un dispositif est propre audit dispositif.

9. Appareil selon la revendication 8, dans lequel les sources d'excitation secondaires desdits dispositifs sont configurées pour fournir ledit signal de référence tour à tour à chacun des dispositifs.

10. Appareil selon l'une des revendications 8 et 9, dans lequel la source d'excitation secondaire et la source d'excitation principale d'un dispositif comprennent au moins une bobine (15) en commun.

11. Appareil de mesure d'une composante d'un champ magnétique comprenant une pluralité de dispositifs (M1-M9) selon l'une des revendications 1 à 7 agencés en réseau, comprenant une source d'excitation secondaire (19x, 19y, 19z) commune auxdits dispositifs.

12. Appareil selon la revendication 11, dans lequel la source d'excitation secondaire comprend au moins une bobine (19x, 19y, 19z) englobant l'appareil et un générateur de fréquence permettant d'injecter un courant d'amplitude connue dans la bobine.

13. Procédé de mesure d'une composante d'un champ magnétique mettant en œuvre une mesure de l'amplitude d'un signal de sortie à une fréquence d'oscillation d'une source d'excitation et une détermination de la composante du champ magnétique à mesurer à partir d'une pente de résonance caractéristique d'une relation de proportionnalité entre ladite amplitude et la composante du champ magnétique à mesurer, **caractérisé par** les étapes de :

- superposition à un signal de mesure (B1) oscillant à une fréquence d'oscillation principale d'un signal de référence d'amplitude connue (B2) oscillant à une fréquence d'oscillation secondaire différente de la fréquence d'oscillation principale,
- mesure de l'amplitude (V) du signal de sortie à un harmonique de la fréquence d'oscillation secondaire, et
- détermination de ladite pente de résonance à partir de ladite amplitude du signal de sortie à un harmonique de la fréquence d'oscillation secondaire et de l'amplitude connue du signal de référence.

**Patentansprüche**

1. Vorrichtung (10) zur Messung einer Komponente eines Magnetfeldes mit einem Detektor (3, 13, 14), der dazu ausgelegt ist, die Amplitude eines Ausgangssignals bei einer Schwingungsfrequenz einer Anregungsquelle zu messen und daraus die zu messende Komponente des Magnetfeldes ausgehend von dem Wert eines Resonanzgefälles abzuleiten, das für eine Proportionalitätsbeziehung zwischen der Amplitude und der zu messenden Komponente des Magnetfeldes charakteristisch ist,
**dadurch gekennzeichnet, dass**
sie eine Hauptanregungsquelle enthält, die ein Messsignal liefert (B1), das mit einer Hauptschwingungsfrequenz schwingt, sowie eine sekundäre Anregungsquelle, die ein Referenzsignal mit bekannter Amplitude (B2) liefert, das mit einer sekundären Schwingungsfrequenz schwingt, die sich von der Hauptschwingungsfrequenz unterscheidet, und dass
der Detektor ferner dazu ausgelegt ist, die Amplitude (V) des Ausgangssignals bei einer Harmonischen der sekundären Schwingungsfrequenz zu messen und daraus den Wert des Resonanzgefälles ausgehend von der Amplitude des Ausgangssignals bei einer Harmonischen der sekundären Schwingungsfrequenz und der bekannten Amplitude des Referenzsignals abzuleiten.

2. Vorrichtung nach Anspruch 1, wobei
der Detektor dazu ausgelegt ist, ein von der sekundären Anregungsquelle erzeugtes Referenzmagnetfeld zu bestimmen, indem er eine Transferfunktion der sekundären Anregungsquelle nutzt, die die Amplitude mit der Magnetfeldkomponente in Beziehung setzt.

3. Vorrichtung nach Anspruch 2, wobei
die sekundäre Anregungsquelle zumindest eine Spule (15) und einen Frequenzgenerator (17) zur Einspeisung eines Stroms mit bekannter Amplitude in die Spule enthält.

4. Vorrichtung nach Anspruch 3, wobei
die sekundäre Anregungsquelle eine Vielzahl von Spulen (15x, 15y, 15z; 19x, 19y, 19z) enthält, die jeweils einer Achse der Vorrichtung zugeordnet sind.

5. Vorrichtung nach Anspruch 4, wobei
die sekundäre Anregungsquelle dazu ausgelegt ist, nacheinander auf jeder der genannten Achsen ein Referenzsignal zu liefern.

6. Vorrichtung nach Anspruch 5, wobei
die sekundäre Anregungsquelle dazu ausgelegt ist, gleichzeitig auf jeder der Achsen ein Referenzsignal zu liefern, wobei das an eine Achse gelieferte Referenzsignal eine andere Schwingungsfrequenz aufweist als das an eine andere Achse gelieferte Referenzsignal oder in Phasenquadratur mit dem an eine andere Achse gelieferten Referenzsignal steht.

7. Vorrichtung nach einem der Ansprüche 1 bis 6, ferner enthaltend eine tertiäre Anregungsquelle (15, 18), die ein Kompensationssignal (B'c) mit konstanter Amplitude liefert, das mit der Hauptschwingungsfrequenz schwingt.

**8.** Gerät zum Messen einer Komponente eines Magnetfeldes mit einer Vielzahl von zu einem Array angeordneten Vorrichtungen (M1 - M9) nach einem der Ansprüche 1 bis 7, wobei die sekundäre Anregungsquelle (15, 17, 15x, 15y, 15z) einer Vorrichtung dieser Vorrichtung zu eigen ist.

**9.** Gerät nach Anspruch 8, wobei
die sekundären Anregungsquellen der Vorrichtungen dazu ausgelegt sind, das Referenzsignal nacheinander an jede der Vorrichtungen zu liefern.

**10.** Gerät nach einem der Ansprüche 8 und 9, wobei
die sekundäre Anregungsquelle und die Hauptanregungsquelle einer Vorrichtung zumindest eine gemeinsame Spule (15) enthalten.

**11.** Gerät zum Messen einer Komponente eines Magnetfeldes, enthaltend eine Vielzahl von zu einem Array angeordneten Vorrichtungen (M1 - M9) nach einem der Ansprüche 1 bis 7, enthaltend eine sekundäre Anregungsquelle (19x, 19y, 19z), die den Vorrichtungen gemein ist.

**12.** Gerät nach Anspruch 11, wobei
die sekundäre Anregungsquelle zumindest eine Spule (19x, 19y, 19z) enthält, die das Gerät umschließt, sowie einen Frequenzgenerator zur Einspeisung eines Stroms mit bekannter Amplitude in die Spule.

**13.** Verfahren zum Messen einer Komponente eines Magnetfeldes, bei dem eine Messung der Amplitude eines Ausgangssignals bei einer Schwingungsfrequenz einer Anregungsquelle und eine Bestimmung der zu messenden Komponente des Magnetfeldes ausgehend von einem Resonanzgefälle erfolgen, das für eine Proportionalitätsbeziehung zwischen der Amplitude und der zu messenden Komponente des Magnetfeldes charakteristisch ist, **gekennzeichnet durch** die folgenden Schritte:

- Überlagerung eines Messsignals (B1), das mit einer Hauptschwingungsfrequenz schwingt, mit einem Referenzsignal bekannter Amplitude (B2), das mit einer von der Hauptschwingungsfrequenz verschiedenen sekundären Schwingungsfrequenz schwingt,
- Messen der Amplitude (V) des Ausgangssignals bei einer Harmonischen der sekundären Schwingungsfrequenz und
- Bestimmen des Resonanzgefälles ausgehend von der Amplitude des Ausgangssignals bei einer Harmonischen der sekundären Schwingungsfrequenz und bekannter Amplitude des Referenzsignals.

**Claims**

**1.** Device (10) for measurement of a magnetic field component comprising a detector (3, 13, 14) configured to measure the amplitude of an output signal at an oscillation frequency of an excitation source and to use it to deduce the component of the magnetic field to be measured starting from the value of a resonance gradient characteristic of a proportionality relation between said amplitude and the magnetic field to be measured,
**characterised in that** it comprises a main excitation source outputting a measurement signal (B1) oscillating at a main oscillation frequency and a secondary excitation source outputting a reference signal with a known amplitude (B2) oscillating at a secondary oscillation frequency different from the main oscillation frequency, and **in that** the detector is also configured to measure the amplitude (V) of the output signal at a harmonic of the secondary oscillation frequency and to use it to deduce the value of said resonance gradient starting from said amplitude of the output signal at a harmonic of the secondary oscillation frequency and the known amplitude of the reference signal.

**2.** Device according to claim 1, in which the detector is configured to determine a magnetic reference field generated by the secondary excitation source making use of a transfer function of the secondary excitation source relating the amplitude to the magnetic field.

**3.** Device according to claim 2, in which the secondary excitation source comprises at least one coil (15) and a frequency generator (17) capable of injecting a current with known amplitude into the coil.

**4.** Device according to claim 3, in which the secondary excitation source comprises a plurality of coils (15x, 15y, 15z ; 19x, 19y, 19z) each associated with an axis of the device.

5. Device according to claim 4, in which the secondary excitation source is configured to output a reference signal onto each of said axes, one by one.

6. Device according to claim 5, in which the secondary excitation source is configured to output a reference signal simultaneously on each of said axes, the oscillation frequency of the reference signal output to one axis being different from the oscillation frequency of the reference signal output to another axis, or being in phase quadrature with the reference signal output to another axis.

7. Device according to one of claims 1 to 6, also comprising a tertiary excitation source (15, 18) outputting a constant amplitude compensation signal (B'c) oscillating at the main oscillation frequency.

8. Instrument for measurement of the magnetic field including a plurality of devices (M1-M9) according to one of claims 1 to 7, arranged in a network, in which the secondary excitation source (15, 17, 15x, 15y, 15z) of a device is specific to said device.

9. Instrument according to claim 8, in which the secondary excitation sources of said devices are configured to output said reference signal to each device one by one.

10. Instrument according to one of claims 8 and 9, in which the secondary excitation source and the main excitation source of a device comprise at least one coil (15) in common.

11. Instrument for measurement of the magnetic field comprising a plurality of devices (M1-M9) according to one of claims 1 to 7, arranged in a network, comprising a secondary excitation source (19x, 19y, 19z) common to said devices.

12. Instrument according to claim 11, in which the secondary excitation source includes at least one coil (19x, 19y, 19z) surrounding the instrument and a frequency generator used to inject a current with known amplitude into the coil.

13. Method of measuring a component of a magnetic field making use of a measurement of the amplitude of an output signal at an oscillation frequency of an excitation source and determination of the component of the magnetic field to be measured making use of the value of a resonance gradient characteristic of a proportionality relation between said amplitude and the magnetic field to be measured, **characterised by** steps of:

   - superposition on a measurement signal oscillating at a main oscillation frequency (B1) of a reference signal with known amplitude (B2) oscillating at a secondary oscillation frequency different from the main oscillation frequency,
   - measurement of the amplitude (V) of the output signal at a harmonic of the secondary oscillation frequency, and
   - determination of said resonance gradient starting from said amplitude of the output signal at a harmonic of the secondary oscillation frequency and at a known amplitude of the reference signal.

FIG.1

FIG.2

FIG.3

FIG.4

A cos $(2\pi f_1 + \pi/2)$       A cos $(2\pi f_2)$       A cos $(2\pi f_2 + \pi/2)$

A cos $(2\pi f_5)$       A cos $(2\pi f_1)$       A cos $(2\pi f_3)$

A cos $(2\pi f_4 + \pi/2)$       A cos $(2\pi f_4)$       A cos $(2\pi f_3 + \pi/2)$

FIG.5

$A \cos(2\pi f_1)$

FIG.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CA 760579 A

- EP 2426563 A1

**Littérature non-brevet citée dans la description**

- **J. DUPONT-ROC.** Etude théorique de diverses résonances observables en champ nul sur des atomes ''habillés'' par des photons de radiofréquence. *Le journal de physique,* Février 1971, 135

- **J.C. ALLDRED et al.** Square cross section coils for the production of uniform magnetic fields. *J. Sci. Instrum.,* 1967, vol. 44